(19) Europäisches Patentamt
European Patent Office
Office européen des brevets

(11) **EP 0 918 053 B1**

(12) # FASCICULE DE BREVET EUROPEEN

(45) Date de publication et mention de la délivrance du brevet:
**10.05.2000 Bulletin 2000/19**

(51) Int Cl.[7]: **C07C 281/00**, C07C 281/16, C07D 213/77, A61K 7/13

(21) Numéro de dépôt: **98402751.6**

(22) Date de dépôt: **05.11.1998**

(54) **Nouveaux composés azoïques, utilisation pour la teinture, compositions les contenant et procédés de teinture.**

Azoverbindungen, ihre Verwendung zum Färben, diese enthaltende Zusammensetzungen und Farbverfahren

Azo compounds, their use for dyeing, compositions containing them and dyeing processes

(84) Etats contractants désignés:
**DE ES FR GB IT**

(30) Priorité: **21.11.1997 FR 9714658**

(43) Date de publication de la demande:
**26.05.1999 Bulletin 1999/21**

(73) Titulaire: **L'OREAL**
**75008 Paris (FR)**

(72) Inventeurs:
- **Henrion, Jean-Christophe**
  **93500 Pantin (FR)**
- **Philippe, Michel**
  **91320 Wissous (FR)**

(74) Mandataire: **Casalonga, Axel**
**BUREAU D.A. CASALONGA - JOSSE**
**Morassistrasse 8**
**80469 München (DE)**

(56) Documents cités:
**DE-B- 2 816 350**
**FR-A- 2 132 214**
**FR-A- 2 570 946**
**US-A- 2 185 154**

- **S. PETERSEN ET AL: ANGEW. CHEM. , vol. 67, no. 8, 1955, pages 217-231, XP002078051**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention est relative à de nouveaux composés azoïques du type 2-(4-amino)-phényl-diazènecarboximidamide, leur procédé de synthèse, leur utilisation pour la teinture des fibres kératiniques, aux compositions de teinture les contenant et aux procédés de teinture les mettant en oeuvre.

Il est bien connu d'utiliser dans les compositions capillaires de coloration, soit des colorants dits d'oxydation; soit des colorants directs. Les premiers conduisent à des nuances plus couvrantes et plus tenaces, mais ils présentent l'inconvénient de ne pas être totalement inoffensifs et de nécessiter une oxydation qui s'accompagne généralement d'une dégradation non négligeable des fibres kératiniques. En outre, leur très grande ténacité et leur très grande affinité pour le cheveu implique le plus souvent l'apparition du phénomène de démarcation entre pointes et demi-longueurs teintes et racines non teintes.

Ces problèmes n'apparaissent pas dans le cas des colorants directs. L'utilisation des colorants directs présente de plus l'avantage par rapport aux précurseurs de colorants d'oxydation de diminuer les risques potentiels d'allergie.

Parmi les colorants directs les plus utilisés, figurent les dérivés nitrés benzéniques. Néanmoins, ces colorants nitrés benzéniques sont insuffisamment résistants aux lavages répétés. Pour palier à ce défaut, on a tenté de remplacer les colorants nitrés benzéniques par des colorants anthraquinoniques aminés ou des colorants azoïques. Il a déjà été proposé, notamment dans la demande de brevet FR-A-2 570 946 l'utilisation du colorant azoïque de formule suivante :

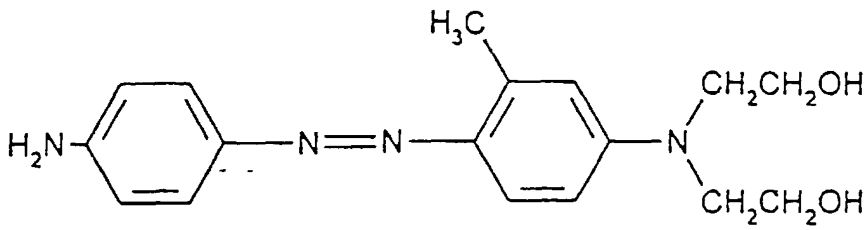

à savoir le 4-amino 2'-méthyl 4'-[N,N-bis-(β-hydroxyéthyl)amino] phényl azobenzène.

Cependant, l'utilisation de ce composé pour la teinture des cheveux n'est pas entièrement satisfaisante, notamment en ce qui concerne la puissance des colorations obtenues.

La demanderesse a donc recherché d'autres colorants azoïques présentant une bonne solubilité dans l'eau, dans les mélanges eau/alcool et plus généralement dans les supports des teintures usuels et qui conduisent, sur les fibres, à des teintures présentant de bonnes propriétés de résistance vis à vis des diverses agressions que peuvent subir les fibres kératiniques et en particulier vis à vis des lavages, de la lumière, des intempéries et de la transpiration.

C'est à la suite de ces recherches que la demanderesse a découvert des composés azoïques du type 2-(4-amino)-phényl-diazènecarboximidamide de formule (I) définie ci-après.

Cette découverte est à la base de la présente invention.

La présente invention a donc pour premier objet de nouveaux composés azoïques de formule (I) suivante, et leurs sels d'addition avec un acide :

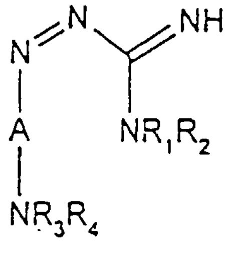

(I)

dans laquelle :

- $R_1$ et $R_2$, indépendamment l'un de l'autre, représentent un atome d'hydrogène ; un radical alkyle en $C_1$-$C_6$ un cycloalkyle à 5 ou 6 chaînons ; un cycle aromatique à 5 ou 6 chaînons ; un cycle aromatique à 5 ou 6 chaînons

substitué par un radical alkyle en $C_1$-$C_6$, un atome d'halogène, un radical amino, hydroxyle ou alcoxy en $C_1$-$C_6$ ; ou forment conjointement avec l'atome d'azote auquel ils sont liés, un hétérocycle comportant de 4 à 8 chaînons, ledit cycle pouvant être interrompu par un ou plusieurs hétéroatomes choisis parmi le soufre, l'azote ou l'oxygène ;

- $R_3$ et $R_4$, indépendamment l'un de l'autre, représentent un atome d'hydrogène, un radical alkyle en $C_1$-$C_6$, un radical monohydroxyalkyle en $C_1$-$C_6$ ou un radical polyhydroxyalkyle en $C_2$-$C_6$ ; et
- A est un noyau aromatique de 5 à 6 chaînons pouvant être interrompu par un ou plusieurs hétéroatomes choisis parmi le soufre, l'azote ou l'oxygène, ou accolé à un autre cycle aromatique de 5 à 6 chaînons.

Lorsqu'ils sont utilisés pour la teinture des fibres kératiniques, les composés de formule (I) conformes à l'invention présentent une bonne solubilité dans l'eau, dans les mélanges eau/alcool et plus généralement dans les supports de teintures usuels, et permettent d'obtenir des colorations présentant une nuance orange cuivrée très intense et résistant bien aux différents traitements que peuvent subir les fibres tels que les lavages, la lumière, les intempéries et la transpiration. Les colorations orange cuivré obtenues en mettant en oeuvre les composés de formule (I) conformes à l'invention sont en particulier plus puissantes que celles obtenues dans les mêmes conditions avec le 4-amino 2'-méthyl 4'-[N,N'-bis-(β-hydroxyéthyl)amino] phényl azobenzène mentionné précédemment.

Dans la formule (I) ci-dessus, les radicaux alkyle, monohydroxyalkyle et polyhydroxyalkyle peuvent être linéaires ou ramifiés.

Parmi les radicaux alkyle en $C_1$-$C_6$ des composés de formule (I) ci-dessus, on peut notamment citer les radicaux méthyle, éthyle, n-propyle, isopropyle, n-butyle, isobutyle, tert-butyle, n-pentyle, isopentyle, n-hexyle et isohexyle.

Parmi les radicaux monohydroxyalkyle en $C_1$-$C_6$, on peut notamment citer les radicaux hydroxyméthyle, hydroxyéthyle, hydroxypropyle et hydroxybutyle. Parmi les radicaux polyhydroxyalkyle en $C_2$-$C_6$, on peut notamment citer les radicaux dihydroxyéthyle, dihydroxypropyle, trihydroxypropyle, et dihydroxybutyle.

Parmi les radicaux cycloalkyle pouvant être représentés par les radicaux $R_1$ et $R_2$, on peut citer le cyclohexyle et le cyclopentyle.

Parmi les cycles aromatiques pouvant être représentés par les radicaux $R_1$ et $R_2$, on peut par exemple mentionner les cycles phényle ; phényle substitué par un radical alkyle en $C_1$-$C_6$, un atome d'halogène, un radical amino, hydroxyle ou alcoxy en $C_1$-$C_6$ ; benzyle, pyrimidine, pyridazine et benzimidazole.

Parmi les hétérocycles pouvant être conjointement formés par les radicaux $R_1$ et $R_2$, on peut notamment citer les cycles imidazole, benzotriazole, benzimidazole, pyrrolidine, pipéridine et morpholine.

Parmi les cycles pouvant être représentés par le noyau aromatique A, on peut par exemple citer les cycles à 6 chaînons tels que les cycles phényle, pyrimidine et pyridine, et les cycles à 5 chaînons tels que les cycles pyrrole et pyrazole.

Les radicaux $R_1$, $R_2$, $R_3$ et $R_4$, indépendamment les uns des autres, représentent préférentiellement un atome d'hydrogène ou un radical méthyle.

Parmi les composés de formule (I), on peut notamment citer :

- le 2-(4-amino)-phényl-diazènecarboximidamide,
- le N,N-diméthyl-2-(4-amino)-phényl-diazènecarboximidamide,
- le N,N-diéthyl-2-(4-amino)-phényl-diazènecarboximidamide,
- le N,N-diisopropyl-2-(4-amino)-phényl-diazènecarboximidamide,
- le N,N-dibutyl-2-(4-amino)-phényl-diazènecarboximidamide,
- le 2-(5-amino)-pyridin-2-yl-diazènecarboximidamide,

et leurs sels d'addition avec un acide.

Les composés de formule (I) conformes à l'invention peuvent être facilement obtenus, selon des méthodes bien connues de l'état de la technique consistant, dans une première étape, à effectuer en milieu solvant, une réaction de réduction sur un composé de formule (III) suivante :

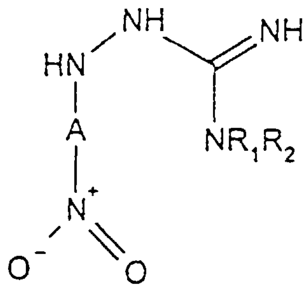

(III)

dans laquelle A, $R_1$ et $R_2$ peuvent prendre les mêmes significations que celles indiquées ci-dessus pour les composés de formule (I), pour obtenir un composé de type 2-(4-amino)-phényl-hydrazinecarboximidamide de formule (II) suivante :

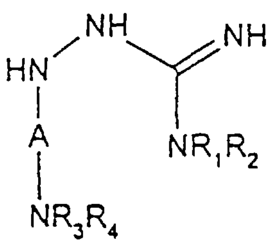

(II)

dans laquelle A, $R_1$, $R_2$, $R_3$ et $R_4$ ont les mêmes significations que celles indiquées ci-dessus pour les composés de formule (I), qui, dans une seconde étape est oxydé en milieu solvant pour donner le composé de formule (I) correspondant.

Selon une variante de ce procédé de synthèse, et lorsque dans les composés de formule (I) l'un au moins des radicaux $R_3$ et $R_4$ est différent d'un atome d'hydrogène, le procédé comporte alors une étape intermédiaire de substitution de l'amine selon les méthodes classiques connues.

Lorsque la synthèse est terminée, les composés de formule (I) conformes à l'invention peuvent, le cas échéant, être récupérés par des méthodes bien connues de l'état de la technique telles que la cristallisation ou la distillation.

Le ou les solvants utilisés lors des étapes 1 et 2 ci-dessus sont de préférence choisis parmi l'eau, les alcanols en $C_1$-$C_4$ tels que le méthanol, l'éthanol, l'isopropanol, et leurs mélanges.

La réaction de réduction utilisée lors de la première étape est de préférence une hydrogénation catalytique, le catalyseur étant par exemple du Palladium sur charbon.

Les agents oxydants pouvant être utilisés lors de la deuxième étape sont de préférence choisis parmi l'iodate de sodium, le periodate de sodium, l'oxygéné de l'air, le monoperoxyde phtalate de magnésium, le peroxyde d'hydrogène, le peroxyde d'hydrogène en présence de sels métalliques tels que par exemple le diacétate de manganèse, le ferricyanure de potassium, l'oxyde d'argent et le chlorure ferrique.

Le pH du milieu de synthèse utilisé lors de la première et de la seconde étape n'est pas critique, il est de préférence compris entre 4 et 10 environ. Il peut être ajusté au moyen des agents alcalinisants ou acidifiants bien connus de l'état de la technique.

La température des réactions réalisées aux étapes 1 et 2 est généralement comprise entre 0 et 60°C environ.

Un autre objet de l'invention est l'utilisation des composés de formule (I) conforme à l'invention à titre colorants pour la teinture des fibres kératiniques, et en particulier des fibres kératiniques humaines telles que les cheveux.

Les composés intermédiaires de formule (II) ci-dessus peuvent également être utilisés à titre de colorants, et en particulier à titre de colorants auto-oxydables (i.e. ne nécessitant pas l'utilisation d'un agent oxydant pour conduire à une coloration) pour la teinture des fibres kératiniques, en particulier des fibres kératiniques humaines telles que les cheveux.

Parmi les composés de formule (II), on peut notamment citer :

- le 2-(4-amino)-phényl-hydrazinecarboximidamide,
- le N,N-diméthyl-2-(4-amino)-phényl-hydrazinecarboximidamide,

- le N,N-diéthyl-2-(4-amino)-phényl-hydrazinecarboximidamide,
- le N,N-diisopropyl-2-(4-amino)-phényl-hydrazinecarboximidamide,
- le N,N-dibutyl-2-(4-amino)-phényl-hydrazinecarboximidamide,
- le 2-(5-amino)-pyridin-2-yl-hydrazinecarboximidamide,

et leurs sels d'addition avec un acide.

Certains composés de formule (II) ci-dessus sont nouveaux en soi et constituent à ce titre un autre objet de l'invention. Ces nouveaux composés, ainsi que leurs sels d'addition avec un acide répondent à la formule (II') suivante :

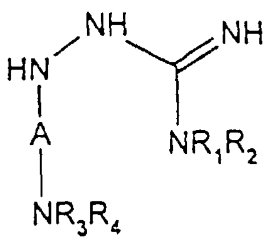

(II')

dans laquelle A, $R_1$, $R_2$, $R_3$ et $R_4$ ont les mêmes significations que celles indiquées ci-dessus pour les composés de formule (I), sous réserve qu'au moins un des radicaux $R_1$, $R_2$, $R_3$ et $R_4$ soit différent d'un atome d'hydrogène.
Le 2-(4-amino)-phényl-hydrazinecarboximidamide (Composé de formule (II') dans lequel $R_1$, $R_2$, $R_3$ et $R_4$ représenteraient simultanément un atome d'hydrogène) est mentionné dans un article de Petersen S. et Coll. (Angew. Chem., 67(1955), pages 217-231) relatifà des fongicides dérivés de quinone.

Parmi les composés de formule (II') conformes à l'invention , on peut notamment citer :

- le N,N-diméthyl-2-(4-amino)-phényl-hydrazinecarboximidamide,
- le N,N-diéthyl-2-(4-amino)-phényl-hydrazinecarboximidamide,
- le N,N-diisopropyl-2-(4-amino)-phényl-hydrazinecarboximidamide,
- le N,N-dibutyl-2-(4-amino)-phényl-hydrazinecarboximidamide,
- le 2-(5-amino)-pyridin-2-yl-hydrazinecarboximidamide,

et leurs sels d'addition avec un acide.

L'invention a également pour objet une composition pour la teinture des fibres kératiniques et en particulier des fibres kératiniques humaines telles que les cheveux, caractérisée par le fait qu'elle comprend, dans un milieu approprié pour la teinture, au moins un composé de formule (I) conforme à l'invention et/ou au moins un composé de formule (II) conforme à l'invention.

Le ou les composés de formule (I) et/ou le ou les composés de formule (II) représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des'fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de

potassium et les composés de formule (IV) suivante :

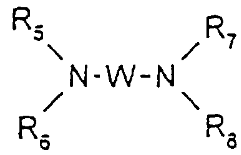

(IV)

dans laquelle W est un reste propylène éventuellement substitué par un groupement hydroxyle ou un radical alkyle en $C_1$-$C_6$ ; $R_5$, $R_6$, $R_7$ et $R_8$, identiques ou différents, représentent un atome d'hydrogène, un radical alkyle en $C_1$-$C_6$ ou hydroxyalkyle en $C_1$-$C_6$.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Afin notamment de faciliter l'oxydation des composés de formule (II) conformes à l'invention lorsqu'ils sont présents dans la composition tinctoriale, ou lorsque celle-ci renferme au moins un précurseur de colorant d'oxydation, ladite composition peut en outre renfermer un ou plusieurs agents oxydants. Ces agents oxydants peuvent notamment être choisis parmi le peroxyde d'hydrogène, le peroxyde d'urée, les bromates de métaux alcalins, les persels tels que les perborates et persulfates, et les enzymes telles que les peroxydases et les oxydo-réductases à deux électrons. L'utilisation du peroxyde d'hydrogène ou des enzymes est particulièrement préférée.

Ainsi que cela a été mentionné précédemment la composition tinctoriale conforme à l'invention peut en outre renfermer une ou plusieurs précurseurs de colorants d'oxydation, c'est à dire une ou plusieurs bases d'oxydation et/ou un ou plusieurs coupleurs. Les bases d'oxydation peuvent notamment être choisies parmi les paraphénylènediamines, les para-aminophénols, les orthophénylènediamines et les bases hétérocycliques telles que par exemple les dérivés pyridiniques, les dérivés pyrimidiniques, les dérivés pyrazoliques, et les dérivés pyrazolopyrimidiniques. Les coupleurs peuvent notamment être choisis parmi les métaphénylènediamines, les méta-aminophénols, les métadiphénols, les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Lorsqu'elles sont présentes, la ou les bases d'oxydation représentent de préférence de 0,0005 à 12 % en poids environ du poids total de la composition tinctoriale conforme à l'invention, et encore plus préférentiellement de 0,005 à 8 % en poids environ de ce poids.

Lorsqu'ils sont présents, le ou les coupleurs représentent de préférence de 0,0001 à 10 % en poids environ du poids total de la composition tinctoriale prête à l'emploi et encore plus préférentiellement de 0,005 à 8 % en poids environ de ce poids.

La composition tinctoriale selon l'invention peut aussi contenir un ou plusieurs colorants directs qui peuvent notamment être choisis parmi les colorants azoïques différents de ceux de l'invention, anthraquinoniques et les dérivés nitrés de la série benzénique, notamment pour modifier les nuances ou les enrichir en reflets.

D'une manière générale, les sels d'addition avec un acide utilisables dans le cadre des compositions tinctoriales de l'invention (composés de formule (I), (II) et (II'), bases d'oxydation additionnelles et coupleurs) sont notamment choisis parmi les chlorhydrates, les bromhydrates, les sulfates, les citrates, les succinates, les tartrates, les lactates et les acétates.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, après quoi on rince, on lave éventuellement

au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, et lorsque la composition tinctoriale conforme à l'invention renferme au moins un composé de formule (II) et au moins un agent oxydant, le procédé comporte une étape préliminaire consistant à stocker sous forme séparée, d'une part, une composition (A) comprenant, dans un milieu approprié pour la teinture, au moins un composé de formule (II) telle que définie précédemment et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant e limiter la portée.

**EXEMPLES DE PREPARATION**

**<u>EXEMPLE DE PREPARATION 1</u> : Synthèse du chlorhydrate du 2-(4-amino)-phényl-diazènecarboximidamide**

Dans un ballon de 500 ml, on a placé 10 g de 2-(4-amino)-phénylhydrazinecarboximidamide dans 250 ml de méthanol à température ambiante. Puis, on a ajouté 20 g d'oxyde d'argent et agité le mélange pendant 30 minutes à température ambiante. Puis, on a filtré le mélange réactionnel sur verre fritté et on y a ajouté 10 ml d'acide chlorhydrique 5,4M dans l'éthanol absolu, puis on a précipité l'azoïque attendu par ajout de 1000 ml d'éther diisopropylique. Par filtration sur verre fritté et lavage à l'éther éthylique, on isole 7,2 g de dichlorhydrate du 2-(4-amino)-phényl-diazènecarboximidamide qui a précipité sous forme d'un solide rouge partiellement hydraté ; ce composé a fondu avec décomposition à 199°C et l'analyse élémentaire calculée était :

| % | C | H | N | Cl |
|---|---|---|---|---|
| Calculé | 35,61 | 4,70 | 29,66 | 30,03 |
| Trouvé | 34,11 | 4,93 | 27,46 | 30,72 |

**<u>EXEMPLE DE PREPARATION 2</u> : Synthèse du chlorhydrate du N,N-diméthyl-2-(4-amino)-phényl-diazènecarboximidamide**

Ce composé a été préparé et purifié selon le mode opératoire décrit ci-dessus pour l'exemple 1.
A partir de 7 g de N,N-diméthyl-2-(4-amino)-phényl hydrazinecarboximidamide et de 14 g d'oxyde d'argent, on a isolé 4,5 g de chlorhydrate du 2-(4-amino)-phényl-diazènecarboximidamide sous forme d'un solide rouge ; ce composé a fondu avec décomposition à 246,6°C et l'analyse élémentaire calculée était :

| % | C | H | N | Cl |
|---|---|---|---|---|
| Calculé | 47,48 | 6,20 | 30,76 | 15,57 |
| Trouvé | 46,17 | 6,32 | 30,04 | 14,08 |

**<u>EXEMPLE DE PREPARATION 3</u> : Synthèse du dichlorhydrate du N,N-diméthyl-2-(4-amino)-phényl-hydrazine-carboximidamide**

Dans un réacteur de 250 ml on a placé 4,0 g de N,N-diméthyl-2-(4-nitro)-phényl-hydrazinecarboximidamide dans 100 ml de méthanol Puis, 0,4 g de palladium sur charbon (humide à 50% et actif à 10%) ont été introduits. Le mélange a été hydrogéné pendant 5 minutes sous 2 bars de pression, puis filtré sur verre fritté. La solution a ensuite été acidifiée par 4 ml d'éthanol chlorhydrique avant d'être précipitée par ajout de 400 ml d'éther diisopropylique. On a isolé 3,5 g de dichlorhydrate du N,N-diméthyl-2-(4-amino)-phényl-hydrazine-carboximidamide sous forme d'un solide blanc ; ce composé a fondu avec décomposition à 185,5°C et l'analyse élémentaire calculée était :

| % | C | H | N | Cl |
|---|---|---|---|---|
| Calculé | 40,61 | 6,44 | 26,31 | 26,18 |
| Trouvé | 39,76 | 6,61 | 25,20 | 26,64 |

**EXEMPLE DE PREPARATION 4 : Synthèse du dichlorhydrate du 2-(4-amino)-phényl-hydrazinecarboximidamide**

Dans un réacteur, on a placé 13,8 g de 2-(4-nitro)-phényl-hydrazinecarboximidamide dans 400 ml d'un mélange méthanol/N,N-diméthylformamide 95/5. Puis, 1,4 g de palladium sur charbon (humide à 50% et actif à 10%) ont été introduits. Le mélange a été hydrogéné pendant 5 minutes sous 2 bars de pression, puis filtré sur verre fritté. La solution a ensuite été acidifiée par 15 ml d'éthanol chlorhydrique avant d'être précipitée par 1000 ml d'éther diisopropylique. On a isolé 6,5 g de dichlorhydrate du 2-(4-amino)-phényl-hydrazinecarboximidamide sous forme d'un solide blanc ; ce composé a fondu avec décomposition à 208°C et l'analyse élémentaire calculée était :

| % | C | H | N | Cl |
|---|---|---|---|---|
| Calculé | 35,31 | 5,50 | 29,41 | 29,78 |
| Trouvé | 35,03 | 5,71 | 27,85 | 30,40 |

**EXEMPLES DE TEINTURES**

**EXEMPLES 1 et 2 COMPARATIFS**

On a préparé les compositions tinctoriales suivantes (teneurs en grammes) :

| **EXEMPLE** | **1(*)** | **2** |
|---|---|---|
| 4-amino 2'-méthyl 4'-[N,N-bis-(β-hydroxyéthyl)amino] phényl azobenzène (Composé ne faisant pas partie de l'invention) | 0,472 | - |
| Dichlorhydrate de 2-(4-amino)-phényl-hydrazinecarboximidamide (composé de formule (II)) | - | 0,099 |
| Support de teinture commun n°1 | (**) | (**) |
| Eau déminéralisée q.s.p. | 100 g | 100 g |

(*) Exemple ne faisant pas partie de l'invention

Il est important de noter que la composition de l'exemple 2 présente une concentration molaire en colorant 4 fois moins importante que celle de l'exemple 1 (respectivement $3,75.10^{-4}$ mole contre $1,5.10^{-4}$ mole).

(**) : Support de teinture commun n°1 ;

- Monoéthyléther d'éthylène glycol 10 g
- Mélange alcool cétylstéarylique / lauryl sulfate de sodium commercialisé sous le nom de SINNOWAX SX ® à par HENKEL 2 g
- Alcool gras linéaire (C13-C15) oxyéthyléné (3 OE) vendu sous la dénomination UKANIL 25 ® par la société PCUK 3 g
- Alcool gras linéaire (C13-C15) oxyéthyléné (7 OE) vendu sous la dénomination UKANIL 43 ® par la société PCUK 2 g
- Bromure de triméthylcétylammonium 1,5 g
- Monoéthanolamine q.s. pH 8

Au moment de l'emploi, chacune des compositions tinctoriales ci-dessus a été appliquée sur des mèches de cheveux gris naturels à 90 % de blancs, à raison de 15 g de composition pour 3 g de cheveux, pendant 20 minutes. Les cheveux ont ensuite été rincés, lavés au shampooing, rincés à nouveau puis séchés.

Les cheveux teints avec les compositions 1 et 2 présentaient la même nuance Jaune orangé.

Afin de déterminer de façon plus précise la montée de la coloration, la couleur des mèches a été évaluée avant et après la teinture dans le système Munsell, au moyen d'un colorimètre MINOLTA CM-2002 ®.

Selon la notation MUNSELL, une couleur est définie par l'expression H V / C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.

La différence entre la couleur de la mèche avant la teinture et la couleur de la mèche après la teinture exprime la puissance de la coloration et a été calculée en appliquant la formule de NICKERSON :

$$\Delta E = 0,4\ C0\Delta H + 6\Delta V + 3\ \Delta C$$

telle que décrite par exemple dans "Couleur, Industrie et Technique" ; pages 14-17; vol. n° 5; 1978.

Dans cette formule, ΔE représente la différence de couleur entre deux mèches, ΔH, ΔV et ΔC représentent la variation en valeur absolue des paramètres H, V et C et $C_0$ représente la pureté de la mèche par rapport à laquelle on désire évaluer la différence de couleur.

Plus la valeur de ΔE est élevée et plus la coloration est puissante

Les résultats sont donnés dans le tableau ci-dessous :

| COMPOSITION | Couleur des cheveux avant la teinture | Couleur des cheveux après la teinture | Puissance de la coloration | | | |
|---|---|---|---|---|---|---|
| | | | ΔH | ΔV | ΔC | ΔE |
| 1 (*) | 3,05 Y 5,7 / 1,9 | 1,85 Y 5,2 / 5,4 | 1,2 | 0,5 | 3,5 | 14,4 |
| 2 | 3,05 Y 5,7 / 1,9 | 1,58 Y 5,3 / 5,2 | 1,5 | 0,4 | 3,3 | 13,4 |

Ces résultats montrent que la composition 2 conforme à l'invention conduit à une coloration aussi puissante que la composition 1 ne faisant pas partie de l'invention et qui contient un colorant ne faisant pas partie de l'invention à une concentration molaire 4 fois plus importante que celle utilisée dans la composition 2.

**EXEMPLES 3 à 6 DE TEINTURE DIRECTE**

On a préparé les compositions tinctoriales suivantes (teneurs en grammes) :

| EXEMPLE | 3 | 4 | 5 | 6 |
|---|---|---|---|---|
| Dichlorhydrate de 2-(4-amino)-phényl-hydrazinecarboximidamide (composé de formule (II)) | 0,396 | 0,099 | | |
| Dichlorhydrate de N,N-diméthyl-2-(4-amino)-phényl-hydrazinecarboximidamide (composé de formule (II)) | - | - | 0,399 | |
| Dichlorhydrate de N,N-diméthyl-2-(4-amino)-phényl-diazènecarboximidamide (composé de formule (I)) | - | - | - | 0,396 |
| Support de teinture commun n°1 | (**) | (**) | (**) | (**) |
| Eau déminéralisée q.s.p. | 100g | 100 g | 100g | 100 g |

(**) : <u>Support de teinture commun n°1</u> :
Il est identique à celui utilisé ci-dessus pour les exemples 1 et 2.
Des mèches de cheveux gris naturels à 90 % de blancs ont été teintes avec chacune des compositions ci-dessus selon le procédé de teinture décrit ci-dessus pour les exemples 1 et 2.

Les nuances obtenues figurent dans le tableau ci-après :

| EXEMPLE | NUANCE OBTENUE |
|---|---|
| 3 | Orangé cuivré |
| 4 | Orangé jaune |
| 5 | Orangé jaune |
| 6 | Orangé rouge |

**<u>EXEMPLES 7 à 15 DE TEINTURE D'OXYDATION</u>**

On a préparé les compositions tinctoriales suivantes (teneurs en grammes) :

| EXEMPLE | 7 | 8 | 9 | 10 | 11 | 12 | 13 | 14 | 15 |
|---|---|---|---|---|---|---|---|---|---|
| Dichlorhydrate de 2-(4-amino)-phényl-hydrazinecarboximidamide (composé de formule (II)) | 0,74 | 0,74 | 0,74 | 0,74 | 0,74 | 0,74 | 0,74 | 0,74 | |
| Dichlorhydrate de N,N-diméthyl-2-(4-amino)-phényl-hydrazinecarboximidamide (composé de formule (II)) | - | - | - | - | - | - | - | - | 0,199 |
| Paraphénylènediamine (Base d'oxydation) | - | - | - | - | - | 0,324 | 0,324 | - | - |
| Para-aminophénol (Base d'oxydation) | - | - | - | - | - | - | - | 0,327 | - |
| Dichlorhydrate de 2,4-diminophénoxyéthanol (Coupleur) | - | 0,723 | - | - | - | 0,723 | - | - | - |
| 2-méthyl 5-N-(β-hydroxyéthyl)amino phénol (Coupleur) | - | - | 0,504 | - | - | - | - | - | - |
| Méta-aminophénol (Coupleur) | - | - | - | 0,327 | - | - | - | 0,327 | - |
| Résorcine (Coupleur) | - | - | - | - | 0,33 | - | 0,33 | - | - |
| Support de teinture commun n°2 | (***) | (***) | (***) | (***) | (***) | (***) | (***) | (***) | (***) |
| Eau déminéralisée q.s.p. | 100 g | 100 g | 100 g | 100 g | 100 g | 100 g | 100 g | 100 g | 100 g |

(**) : Support de teinture commun n°2 :

| | | |
|---|---|---|
| - Alcool oléique polyglycérolé à 2 moles de glycérol | 4,0 | g |
| - Alcool oléique polyglycérolé à 4 moles de glycérol à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 ® par la société AKZO | 7,0 | g |
| - Laurylamino succinamate de diéthylaminopropyle, sel de sodium à 55 % de M.A. | 3,0 | g M.A. |
| - Alcool oléique | 5,0 | g |
| - Diéthanolamide d'acide oléique | 12,0 | g |
| - Propylèneglycol | 3,5 | g |
| - Alcool éthylique | 7,0 | g |
| - Dipropylèneglycol | 0,5 | g |
| - Monométhyléther de propylèneglycol | 9,0 | g |
| - Métabisulfite de sodium à en solution aqueuse à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,0 | g |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris, naturels ou permanentés, à 90 % de blancs pendant 30 minutes. Les mèches ont ensuite été rincés, lavés avec un shampooing standard, rincées à nouveau puis séchées.

Les nuances obtenues figurent dans le tableau ci-après :

| EXEMPLE | NUANCE OBTENUE |
|---|---|
| 7 | Orangé jaune |
| 8 | Brun orangé |
| 9 | Orangé |
| 10 | Orangé rouge |
| 11 | Orangé jaune |

(suite)

| EXEMPLE | NUANCE OBTENUE |
|---|---|
| **12** | Brun noir |
| **13** | Orangé brun |
| **14** | Orangé jaune |
| **15** | Jaune orangé |

## Revendications

1. Composés azoïques de formule (I) suivante, et leurs sels d'addition avec un acide :

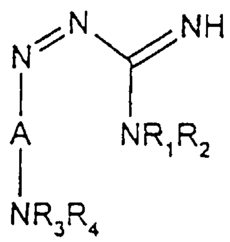

(I)

dans laquelle :

- $R_1$ et $R_2$, indépendamment l'un de l'autre, représentent un atome d'hydrogène ; un radical alkyle en $C_1$-$C_6$ ; un cycloalkyle à 5 ou 6 chaînons ; un cycle aromatique à 5 ou 6 chaînons ; un cycle aromatique à 5 ou 6 chaînons substitué par un radical alkyle en $C_1$-$C_6$, un atome d'halogène, un radical amino, hydroxyle ou alcoxy en $C_1$-$C_6$ ; ou forment conjointement avec l'atome d'azote auquel ils sont liés, un hétérocycle comportant de 4 à 8 chaînons, ledit cycle pouvant être interrompu par un ou plusieurs hétéroatomes choisis parmi le soufre, l'azote ou l'oxygène ;
- $R_3$ et $R_4$, indépendamment l'un de l'autre, représentent un atome d'hydrogène, un radical alkyle en $C_1$-$C_6$, un radical monohydroxyalkyle en $C_1$-$C_6$ ou un radical polyhydroxyalkyle en $C_2$-$C_6$ ; et
- A est un noyau aromatique de 5 à 6 chaînons pouvant être interrompu par un ou plusieurs hétéroatomes choisis parmi le soufre, l'azote ou l'oxygène, ou accolé à un autre cycle aromatique de 5 à 6 chaînons.

2. Composés selon la revendication 1, caractérisés par le fait que le noyau aromatique A est un cycle à 6 chaînons choisi parmi les cycles phényle, pyrimidine et pyridine, ou un cycle à 5 chaînons choisi parmi les cycles pyrrole et pyrazole.

3. Composés selon la revendication 1 ou 2, caractérisés par le fait qu'ils sont choisis parmi :

- le 2-(4-amino)-phényl-diazènecarboximidamide,
- le N,N-diméthyl-2-(4-amino)-phényl-diazènecarboximidamide,
- le N,N-diéthyl-2-(4-amino)-phényl-diazènecarboximidamide,
- le N,N-diisopropyl-2-(4-amino)-phényl-diazènecarboximidamide,
- le N,N-dibutyl-2-(4-amino)-phényl-diazènecarboximidamide,
- le 2-(5-amino)-pyridin-2-yl-diazènecarboximidamide,

et leurs sels d'addition avec un acide.

4. Utilisation des composés de formule (I) tels que définis à l'une quelconque des revendications 1 à 3 à titre de colorants pour la teinture des fibres kératiniques, et en particulier des fibres kératiniques humaines telles que les cheveux.

5. Utilisation des composés de formule (II) suivante, et de leurs sels d'addition avec un acide :

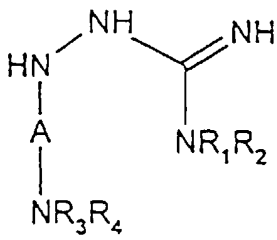

(II)

dans laquelle $R_1$, $R_2$, $R_3$, $R_4$ et A ont les mêmes significations que celles indiquées à la revendication 1, à titre de colorants pour la teinture des fibres kératiniques, en particulier des fibres kératiniques humaines telles que les cheveux.

**6.** Utilisation selon la revendication 5, caractérisée par le fait que les composés de formule (II) sont choisis parmi :

- le 2-(4-amino)-phényl-hydrazinecarboximidamide,
- le N,N-diméthyl-2-(4-amino)-phényl-hydrazinecarboximidamide,
- le N,N-diéthyl-2-(4-amino)-phényl-hydrazinecarboximidamide,
- le N,N-diisopropyl-2-(4-amino)-phényl-hydrazinecarboximidamide,
- le N,N-dibutyl-2-(4-amino)-phényl-hydrazinecarboximidamide,
- le 2-(5-amino)-pyridin-2-yl-hydrazinecarboximidamide,

et leurs sels d'addition avec un acide.

**7.** Composés de formule (II') suivante, et leurs sels d'addition avec un acide :

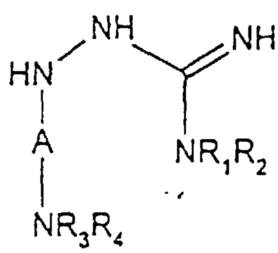

(II')

dans laquelle $R_1$, $R_2$, $R_3$, $R_4$ et A ont les mêmes significations que celles indiquées à la revendication 1, sous réserve qu'au moins un des radicaux $R_1$, $R_2$, $R_3$ et $R_4$ soit différent d'un atome d'hydrogène.

**8.** Composés selon la revendication 7, caractérisés par le fait qu'ils sont choisis parmi:

- le N,N-diméthyl-2-(4-amino)-phényl-hydrazinecarboximidamide,
- le N,N-diéthyl-2-(4-amino)-phényl-hydrazinecarboximidamide,
- le N,N-diisopropyl-2-(4-amino)-phényl-hydrazinecarboximidamide,
- le N,N-dibutyl-2-(4-amino)-phényl-hydrazinecarboximidamide,
- le 2-(5-amino)-pyridin-2-yl-hydrazinecarboximidamide,

et leurs sels d'addition avec un acide.

**9.** Composition pour la teinture des fibres kératiniques et en particulier des fibres kératiniques humaines telles que les cheveux, caractérisée par le fait qu'elle comprend, dans un milieu approprié pour la teinture, au moins un composé de formule (I) tel que défini à l'une quelconque des revendications 1 à 3 et/ou au moins un composé de formule (II) tel que défini à l'une quelconque des revendications 5 et 6.

**10.** Composition selon la revendication 9, caractérisée par le fait que le ou les composés de formule (I) et/ou le ou les composés de formule (II) représentent de 0,0005 à 12 % en poids du poids total de la composition tinctoriale.

**11.** Composition selon la revendication 10, caractérisée par le fait que le ou les composés de formule (I) et/ou le ou les composés de formule (II) représentent de 0,005 à 6 % en poids du poids total de la composition tinctoriale.

**12.** Composition selon l'une quelconque des revendications 9 à 11, caractérisée par le fait que le milieu approprié pour la teinture (ou support) est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**13.** Composition selon l'une quelconque des revendications 9 à 12, caractérisée par le fait qu'elle présente un pH compris entre 3 et 12.

**14.** Composition selon l'une quelconque des revendications 9 à 13, caractérisée par le fait qu'elle renferme un agent oxydant.

**15.** Composition selon la revendication 14, caractérisée par le fait que l'agent oxydant est choisi parmi le peroxyde d'hydrogène, le peroxyde d'urée, les bromates de métaux alcalins, les persels tels que les perborates et persulfates, et les enzymes.

**16.** Composition selon l'une quelconque des revendications 14 à 15, caractérisée par le fait qu'elle renferme une ou plusieurs bases d'oxydation et/ou un ou plusieurs coupleurs.

**17.** Composition selon la revendication 16, caractérisée par le fait que la ou les bases d'oxydation représentent de 0,0005 à 12 % en poids du poids total de la composition tinctoriale et que le ou les coupleurs représentent de 0,0001 à 10 % en poids du poids total de la composition tinctoriale.

**18.** Composition selon la revendication 17, caractérisée par le fait que la ou les bases d'oxydation représentent de 0,005 à 8 % en poids du poids total de la composition tinctoriale et que le ou les coupleurs représentent de 0,005 à 8 % en poids du poids total de la composition tinctoriale.

**19.** Composition selon l'une quelconque des revendications 9 à 18, caractérisée par le fait que les sels d'addition avec un acide sont choisis parmi les chlorhydrates, les bromhydrates, les sulfates, les citrates, les succinates, les tartrates, les lactates et les acétates.

**20.** Procédé de teinture des fibres kératiniques et en particulier des fibres kératiniques humaines telles que les cheveux, caractérisé par le fait que l'on applique sur les fibres au moins une composition tinctoriale telle que définie à l'une quelconque des revendications 9 à 19 pendant un temps suffisant pour développer la coloration désirée.

**21.** Procédé selon la revendication 20, caractérisé par le fait qu'il comporte une étape préliminaire consistant à stocker sous forme séparée, d'une part, une composition (A) comprenant, dans un milieu approprié pour la teinture, au moins un composé de formule (II) tel que défini à l'une quelconque des revendications 5 et 6 et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

**22.** Dispositif à plusieurs compartiments ou "kit" de teinture, caractérisé par le fait qu'il'comporte un premier compartiment renfermant la composition (A) telle que définie à la revendication 21 et un second compartiment renfermant la composition (B) telle que définie à la revendication 21.

## Patentansprüche

**1.** Azoverbindungen der folgenden Formel (I) und die Additionssalze dieser Verbindungen mit einer Säure:

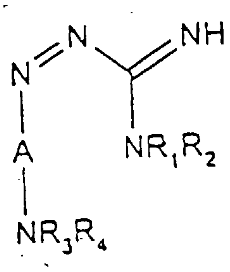

(I),

worin:

- die Gruppen $R_1$ und $R_2$ unabhängig voneinander Wasserstoff, $C_{1-6}$-Alkyl, eine 5- oder 6-gliedrige Cycloalkylgruppe, einen 5- oder 6-gliedrigen aromatischen Ring oder einen 5- oder 6-gliedrigen aromatischen Ring, der mit einer $C_{1-6}$-Alkylgruppe, einem Halogenatom, einer Aminogruppe, einer Hydroxygruppe oder einer $C_{1-6}$-Alkoxygruppe substituiert ist, bedeuten oder gemeinsam mit dem Stickstoffatom, an das sie gebunden sind, einen 4- bis 8-gliedrigen Heterocyclus bilden, wobei der Ring durch ein oder mehrere Heteroatome, die unter Schwefel, Stickstoff oder Sauerstoff ausgewählt sind, unterbrochen sein kann;
- die Gruppen $R_3$ und $R_4$ unabhängig voneinander Wasserstoff, $C_{1-6}$-Alkyl, $C_{1-6}$-Monohydroxyalkyl oder $C_{2-6}$-Polyhydroxyalkyl bedeuten; und
- die Gruppe A ein 5- bis 6-gliedriger aromatischer Ring ist, der durch ein oder mehrere Heteroatome, die unter Schwefel, Stickstoff oder Sauerstoff ausgewählt sind, unterbrochen sein kann oder mit einem weiteren 5- bis 6-gliedrigen aromatischen Ring verbunden sein kann.

**2.** Verbindungen nach Anspruch 1, dadurch gekennzeichnet, daß der aromatische Ring A ein 6-gliedriger Ring, der unter Phenyl, Pyrimidin und Pyridin ausgewählt ist, oder ein 5-gliedriger Ring ist, der unter Pyrrol und Pyrazol ausgewählt ist.

**3.** Verbindungen nach Anspruch 1 oder 2, dadurch gekennzeichnet, daß sie ausgewählt sind unter:

- 2-(4-Amino)-phenyl-diazencarboximidamid,
- N,N-Dimethyl-2-(4-amino)-phenyl-diazencarboximidamid,
- N, N-Diethyl-2-(4-Amino)-phenyl-diazencarboximidamid,
- N,N-Diisopropyl-2-(4-amino)-phenyl-diazencarboximidamid,
- N,N-Dibutyl-2-(4-amino)-phenyl-diazencarboximidamid,
- 2-(5-Amino)-pyridin-2-yl-diazencarboximidamid

und den Additionssalzen dieser Verbindungen mit einer Säure.

**4.** Verwendung von Verbindungen der oben definierten Formel (I) nach einem der Ansprüche 1 bis 3 als Farbstoffe zum Färben von Keratinfasern und insbesondere von menschlichen Keratinfasern, wie dem Haar.

**5.** Verwendung von Verbindungen der folgenden Formel (II) und der Additionssalze dieser Verbindungen mit einer Säure:

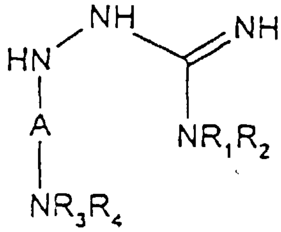

(II),

worin die Gruppen $R_1$, $R_2$, $R_3$, $R_4$ und A die in Anspruch 1 angegebenen Bedeutungen aufweisen, als Farbstoffe zum Färben von Keratinfasern und insbesondere menschlichen Keratinfasern, wie dem Haar.

6. Verwendung nach Anspruch 5, dadurch gekennzeichnet, daß die Verbindungen der Formel (II) ausgewählt sind unter:

- 2-(4-Amino)-phenyl-hydrazincarboximidamid,
- N,N-Dimethyl-2- (4-amino)-phenyl-hydrazincarboximidamid,
- N/N-Diethyl-2-(4-amino)-phenyl-hydrazincarboximidamid,
- N,N-Diisopropyl-2-(4-amino)-phenyl-hydrazincarboximidamid,
- N,N-Dibutyl-2-(4-amino)-phenyl-hydrazincarboximidamid,
- 2-(5-Amino) -pyridin-2-yl-hydrazincarboximidamid

und den Additionssalzen dieser Verbindungen mit einer Säure.

7. Verbindungen der folgenden Formel (II') und die Additionssalze dieser Verbindungen mit einer Säure:

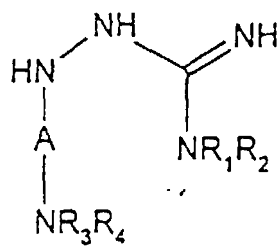

(II')

worin die Gruppen $R_1$, $R_2$, $R_3$, $R_4$ und A die in Anspruch 1 angegebenen Bedeutungen aufweisen, mit der Maßgabe, daß mindestens eine der Gruppen $R_1$, $R_2$, $R_3$ und $R_4$ von Wasserstoff verschieden ist.

8. Verbindungen nach Anspruch 7, dadurch gekennzeichnet, daß sie ausgewählt sind unter:

- N,N-Dimethyl-2-(4-amino)-phenyl-hydrazincarboximidamid,
- N,N-Diethyl-2-(4-amino)-phenyl-hydrazincarboximidamid,
- N,N-Diisopropyl-2-(4-amino)-phenyl-hydrazincarboximidamid,
- N,N-Dibutyl-2-(4-amino)-phenyl-hydrazincarboximidamid,
- 2-(5-Amino)-pyridin-2-yl-hydrazincarboximidamid

und den Additionssalzen dieser Verbindungen mit einer Säure.

9. Zusammensetzung zum Färben von Keratinfasern und insbesondere menschlichen Keratinfasern, wie dem Haar, dadurch gekennzeichnet, daß sie in einem zum Färben geeigneten Medium mindestens eine Verbindung der Formel (I) nach einem der Ansprüche 1 bis 3 und/oder mindestens eine Verbindung der Formel (II) nach einem der Ansprüche 5 und 6 enthält.

10. Zusammensetzung nach Anspruch 9, dadurch gekennzeichnet, daß die Verbindung(en) der Formel (I) und/oder die Verbindung(en) der Formel (II) 0,0005 bis 12 Gew.-% des Gesamtgewichts der Färbemittelzusammensetzung ausmachen.

11. Zusammensetzung nach Anspruch 10, dadurch gekennzeichnet, daß die Verbindung(en) der Formel (I) und/oder die Verbindung(en) der Formel (II). 0,005 bis 6 Gew.-% des Gesamtgewichts der Färbemittelzusammensetzung ausmachen.

12. Zusammensetzung nach einem der Ansprüche 9 bis 11, dadurch gekennzeichnet, daß das zum Färben geeignete Medium (oder der Träger) aus Wasser oder einem Gemisch von Wasser und mindestens einem organischen Lösungsmittel besteht, das unter den niederen $C_{1-4}$-Alkanolen, Glycerin, Glykolen und Glykolethern, aromatischen Alkoholen, analogen Produkten und deren Gemischen ausgewählt ist.

13. Zusammensetzung nach einem der Ansprüche 9 bis 12, dadurch gekennzeichnet, daß sie einen pH-Wert im Bereich von 3 bis 12 aufweist.

**14.** Zusammensetzung nach einem der Ansprüche 9 bis 13, dadurch gekennzeichnet, daß sie ein Oxidationsmittel enthält.

**15.** Zusammensetzung nach Anspruch 14, dadurch gekennzeichnet, daß das Oxidationsmittel unter Wasserstoffperoxid, Harnstoffperoxid, Alkalimetallbromaten, Salzen von Persäuren, wie Perboraten und Persulfaten, und Enzymen ausgewählt ist.

**16.** Zusammensetzung nach einem der Ansprüche 14 bis 15, dadurch gekennzeichnet, daß sie eine oder mehrere Oxidationsbasen und/oder einen oder mehrere Kuppler enthält.

**17.** Zusammensetzung nach Anspruch 16, dadurch gekennzeichnet, daß die Oxidationsbase(n) 0,0005 bis 12 Gew.-% des Gesamtgewichts der Färbemittelzusammensetzung und der oder die Kuppler 0,0001 bis 10 Gew.-% des Gesamtgewichts der Färbemittelzusammensetzung ausmachen.

**18.** Zusammensetzung nach Anspruch 17, dadurch gekennzeichnet, daß die Oxidationsbase(n) 0,005 bis 8 Gew.-% des Gesamtgewichts der Färbemittelzusammensetzung und der oder die Kuppler 0,005 bis 8 % des Gesamtgewichts der Färbemittelzusammensetzung ausmachen.

**19.** Zusammensetzung nach einem der Ansprüche 9 bis 18, dadurch gekennzeichnet, daß die Additionssalze mit einer Säure unter den Hydrochloriden, Hydrobromiden, Sulfaten, Citraten, Succinaten, Tartraten, Lactaten und Acetaten ausgewählt sind.

**20.** Verfahren zum Färben von Keratinfasern und insbesonderemenschlichen Keratinfasern, wie dem Haar, dadurch gekennzeichnet, daß auf die Fasern mindestens eine Färbemittelzusammensetzung nach einem der Ansprüche 9 bis 12 während einer Zeitspanne aufgebracht wird, die ausreicht, um die gewünschte Färbung zu entwickeln.

**21.** Verfahren nach Anspruch 20, dadurch gekennzeichnet, daß es einen vorbereiteten Schritt umfaßt, der darin besteht, eine Zusammensetzung (A), die in einem zum Färben geeigneten Medium mindestens eine Verbindung der Formel (II) nach einem der Ansprüche 5 und 6 enthält, und eine Zusammensetzung (B), die in einem zum Färben geeigneten Medium mindestens ein Oxidationsmittel enthält, getrennt voneinander aufzubewahren und diese dann bei der Anwendung zu vermischen, bevor das Gemisch auf die Keratinfasern aufgebracht wird.

**22.** Vorrichtung mit mehreren Abteilungen oder "Kit" zum Färben, dadurch gekennzeichnet, daß sie eine erste Abteilung, die die Zusammensetzung (A) nach Anspruch 21 enthält, und eine zweite Abteilung aufweist, die die Zusammensetzung (B) nach Anspruch 21 enthält.

## Claims

**1.** An azo compound of formula (I) below, and the addition salt thereof with an acid:

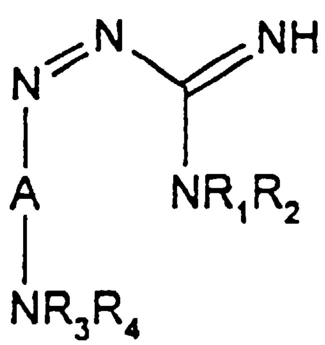

(I)

in which:

- $R_1$ and $R_2$, independently of each other, represent a hydrogen atom; a $C_1$-$C_6$ alkyl radical; a 5- or 6-membered

cycloalkyl radical; a 5- or 6-membered aromatic ring; a 5- or 6-membered aromatic ring substituted with a $C_1$-$C_6$ alkyl radical, a halogen atom or an amino, hydroxyl or $C_1$-$C_6$ alkoxy radical; or form, together with the nitrogen atom to which they are attached, a 4- to 8-membered heterocycle, it being possible for the said ring to be interrupted by one or more hetero atoms chosen from sulfur, nitrogen and oxygen;

- $R_3$ and $R_4$, independently of each other, represent a hydrogen atom, a $C_1$-$C_6$ alkyl radical, a $C_1$-$C_6$ monohydroxyalkyl radical or a $C_2$-$C_6$ polyhydroxyalkyl radical; and
- A is a 5- to 6-membered aromatic ring which can be interrupted by one or more hetero atoms chosen from sulfur, nitrogen and oxygen, or fused to another 5- to 6-membered aromatic ring.

**2.** The compound as claimed in claim 1, wherein the aromatic ring A is a 6-membered ring chosen from phenyl, pyrimidine and pyridine rings, or a 5-membered ring chosen from pyrrole and pyrazole rings.

**3.** The compound as claimed in claim 1 or 2, wherein it is chosen from:

- 2-(4-amino)phenyldiazinecarboximidamide,
- N,N-dimethyl-2-(4-amino)phenyldiazinecarboximidamide,
- N, N-diethyl-2-(4-amino)phenyldiazinecarboximidamide,
- N,N-diisopropyl-2- (4-amino)phenyldiazinecarboximidamide,
- N,N-dibutyl-2-(4-amino)phenyldiazinecarboximidamide,
- 2-(5-amino)-2-pyridyldiazinecarboximidamide,

and the addition salt thereof with an acid.

**4.** The use of the compound of formula (I) as defined in any one of claims 1 to 3, as a dye for dyeing keratin fibers, and in particular human keratin fibers such as the hair.

**5.** The use of the compound of formula (II) below, and the addition salt thereof with an acid:

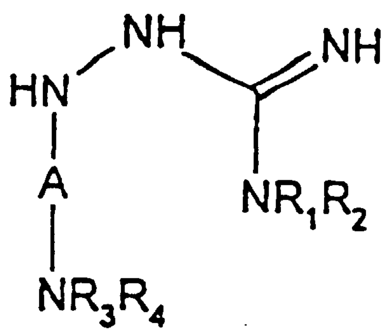

(II)

in which $R_1$, $R_2$, $R_3$, $R_4$ and A have the same meanings as those indicated in claim 1, as a dye for dyeing keratin fibers, in particular human keratin fibers such as the hair.

**6.** Use as claimed in claim 5, wherein the compound of formula (II) is chosen from:

- 2-(4-amino)phenylhydrazinecarboximidamide,
- N,N-dimethyl-2-(4-amino)phenylhydrazinecarboximidamide,
- N,N-diethyl-2-(4-amino)phenylhydrazinecarboximidamide,
- N,N-diisopropyl-2-(4-amino)phenylhydrazinecarboximidamide,
- N,N-dibutyl-2-(4-amino)phenylhydrazinecarboximidamide,
- 2-(5-amino)-2-pyridylhydrazinecarboximidamide,

and the addition salt thereof with an acid.

**7.** The compound of formula (II') below, and the addition salt thereof with an acid:

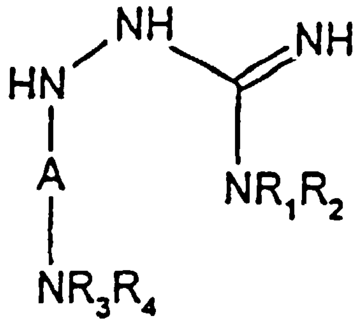

(II')

in which $R_1$, $R_2$, $R_3$, $R_4$ and A have the same meanings as those indicated in claim 1, with the proviso that at least one of the radicals $R_1$, $R_2$, $R_3$ and $R_4$ is other than a hydrogen atom.

**8.** The compound as claimed in claim 7, wherein it is chosen from:

- N,N-dimethyl-2-(4-amino)phenylhydrazinecarboximidamide,
- N,N-diethyl-2-(4-amino)phenylhydrazinecarboximidamide,
- N,N-diisopropyl-2-(4-amino)phenylhydrazinecarboximidamide,
- N,N-dibutyl-2-(4-amino)phenylhydrazinecarboximidamide,
- 2-(5-amino)-2-pyridylhydrazinecarboximidamide,

and the addition salt thereof with an acid.

**9.** A composition for dyeing keratin fibers, and in particular human keratin fibers such as the hair, wherein it comprises, in a medium which is suitable for dyeing, at least one compound of formula (I) as defined in any one of claims 1 to 3 and/or at least one compound of formula (II) as defined in either of claims 5 and 6.

**10.** The composition as claimed in claim 9, wherein the compound(s) of formula (I) and/or the compound(s) of formula (II) represent(s) from 0.0005 to 12% by weight relative to the total weight of the dye composition.

**11.** The composition as claimed in claim 10, wherein the compound(s) of formula (I) and/or the compound(s) of formula (II) represent(s) from 0.005 to 6% by weight relative to the total weight of the dye composition.

**12.** The composition as claimed in any one of claims 9 to 11, wherein the medium which is suitable for dyeing (or the support) consists of water or of a mixture of water and at least one organic solvent chosen from $C_1$-$C_4$ lower alkanols, glycerol, glycols, glycol ethers and aromatic alcohols, similar products and mixtures thereof.

**13.** The composition as claimed in any one of claims 9 to 12, wherein it has a pH of between 3 and 12.

**14.** The composition as claimed in any one of claims 9 to 13, wherein it contains an oxidizing agent.

**15.** The composition as claimed in claim 14, wherein the oxidizing agent is chosen from hydrogen peroxide, urea peroxide, alkali metal bromates, persalts such as perborates and persulfates, and enzymes.

**16.** The composition as claimed in either of claims 14 and 15, wherein it contains one or more oxidation bases and/or one or more couplers.

**17.** The composition as claimed in claim 16, wherein the oxidation base(s) represent(s) from 0.0005 to 12% by weight relative to the total weight of the dye composition and wherein the coupler(s) represent(s) from 0.0001 to 10% by weight relative to the total weight of the dye composition.

**18.** The composition as claimed in claim 17, wherein the oxidation base(s) represent(s) from 0.005 to 8% by weight relative to the total weight of the dye composition and wherein the coupler(s) represent(s) from 0.005 to 8% by weight relative to the total weight of the dye composition.

**19.** The composition as claimed in any one of claims 9 to 18, wherein the addition salts with an acid are chosen from

the hydrochlorides, hydrobromides, sulfates, citrates, succinates, tartrates, lactates and acetates.

**20.** Process for dyeing keratin fibers, and in particular human keratin fibers such as the hair, wherein at least one dye composition as defined in any one of claims 9 to 19 is applied to the fibers for a period which is sufficient to develop the desired colouring.

**21.** The process as claimed in claim 20, wherein it includes a preliminary step which consists in separately storing, on the one hand, a composition (A) comprising, in a medium which is suitable for dyeing, at least one compound of formula (II) as defined in either of claims 5 and 6, and, on the other hand, a composition (B) containing, in a medium which is suitable for dyeing, at least one oxidizing agent, and then in mixing them together at the time of use, before applying this mixture to the keratin fibers.

**22.** Multi-compartment dyeing device or "kit", wherein it includes a first compartment containing composition (A) as defined in claim 21 and a second compartment containing composition (B) as defined in claim 21.